# EUROPEAN PATENT APPLICATION

(11) **EP 3 009 186 A1**
(43) Date of publication of application: **20.04.2016**
(21) Application number: 14189005.3
(22) Date of filing: 15.10.2014
(51) Int. Cl.: B01J 8/04, C07C 1/04, C10L 3/08, B01D 53/047

(54) **A reactor system with means for catalyst protection during trips or shut-down**

(71) Applicant: Haldor Topsøe A/S, 2800 Kgs. Lyngby (DK)
(72) Inventor: Wix, Christian, 2850 Nærum (DK)
(74) Representative: Haldor Topsøe A/S

(57) **Abstract**

A reactor system with means for catalyst protection during trips or shut-down comprises an inlet line (i) for a process gas, a sulfur guard (S), an adsorption vessel (A) containing an adsorbent selective for adsorbing a reactor purge gas and an outlet line (o) for the product stream from a reactor (R). The adsorption vessel (A) is installed either upstream or downstream the sulfur guard (S) in parallel to the normal piping.

## Description

The present invention relates to a reactor system with means for catalyst protection during trips or shut-down. It also relates to a process for shut-down of the reactor system of the invention.

More specifically, the invention is based on installing an adsorption vessel on the inlet line either upstream or downstream a sulfur guard, in parallel to normal piping, to be used in case of trips or shut-down. When the pressure in the system is reduced due to shut-down, then the gas upstream the adsorption vessel will pass through to increase the hydrogen concentration by retaining CH₄ and CO, thereby decreasing the risk of carbon formation in the downstream reactor, which is preferably a methanation reactor. The filling material can e.g. be Sylobead®.

GB patent application 2 223 237 A describes a shut-down process for a Fischer-Tropsch reactor and the reactor itself. More specifically it describes a process for the shut-down of a reactor for the preparation of an at least partly liquid hydrocarbonaceous product by a catalytic reaction of carbon monoxide with hydrogen at an elevated temperature and pressure and using a catalyst, said reactor being provided with cooling means and with means to recycle gas through the catalyst for temperature equalizing of the catalyst. The process comprises the steps of (1) interrupting the feed of synthesis gas, (2) depressurizing the reactor downstream of the catalyst, providing the reactor upstream of the catalyst with inert gas, and (3) cooling the catalyst to ambient conditions.

US patent application 2008/0075986 A1 concerns a fuel cell stack shut-down purge method, more specifically a method for purging hydrogen from a fuel cell stack after fuel cell shut-down. The method includes providing an air stream, providing a temporary nitrogen stream by removing oxygen from the air stream with an adsorbent bed and passing the nitrogen stream through the fuel cell stack. This is done to avoid a loss of carbon substrate and catalyst area, which otherwise will reduce the operating voltage and ultimately limit the life time of the stack.

WO 2005/101556 relates to fuel cell shut-down with steam purging, more specifically to a method and an apparatus for steam purging a solid oxide fuel cell stack. Purging the SOFC stack with steam has a physical flushing effect, removing carbon monoxide containing reformate and free oxygen gas from the anode area, thereby reducing the potential for nickel oxide or nickel carbonyl formation.

The reactor system according to the invention comprises
- an inlet line i for a process gas,
- a sulfur guard S,
- an adsorption vessel A containing an adsorbent selective for adsorbing a reactor purge gas, and
- an outlet line o for the product gas stream from a reactor R,
   wherein the adsorption vessel is installed either upstream or downstream the sulfur guard, in parallel to the normal piping, to be used in case of trips or shut-down.

Advantageously, two adsorption vessels arranged in parallel can be used. This way, one of the vessels can be regenerated while the other is in use.

Regeneration of the adsorption vessel can for instance be done by relieving the pressure to flare.

The adsorption vessel in the system of the present invention is preferably based on the use of pressure swing adsorption (PSA), a technology used to separate a gas species from a mixture of gases under pressure according to the molecular characteristics of the species and its affinity for a given adsorbent material. PSA operates at near-ambient temperatures and differs significantly from e.g. cryogenic distillation techniques of gas separation. Specific adsorptive materials, such as zeolites, activated carbon, molecular sieves etc., are used as a trap, preferentially adsorbing the target gas species at high pressure.

PSA processes rely on the fact that under high pressure, gases tend to be attracted to solid surfaces, or "adsorbed". The higher the pressure, the more gas is adsorbed. When the pressure is reduced, the gas is released, or "desorbed". PSA processes can be used to effectively separate gases in a mixture because different gases tend to be attracted to different solid surfaces more or less strongly. If, for example, a gas mixture such as air is passed under pressure through a vessel containing an adsorbent bed of zeolite that attracts nitrogen more strongly than it does oxygen, then part or all of the nitrogen will remain in the bed, and the gas coming out of the vessel will be enriched in oxygen. When the bed reaches the end of its capacity to adsorb nitrogen, it can be regenerated by reducing the pressure, thereby releasing the adsorbed nitrogen. It is then ready for another cycle of producing oxygenenriched air.

Aside from their ability to discriminate between different gases, adsorbents for PSA systems are usually very porous materials, chosen because of their large surface areas. Typical adsorbents are activated carbon, silica gel, alumina and zeolites. Though the gas adsorbed on these surfaces may consist of a layer only one or at most a few molecules thick, surface areas of several hundred square meters per gram enable the adsorption of a significant portion of the adsorbent's weight in gas.

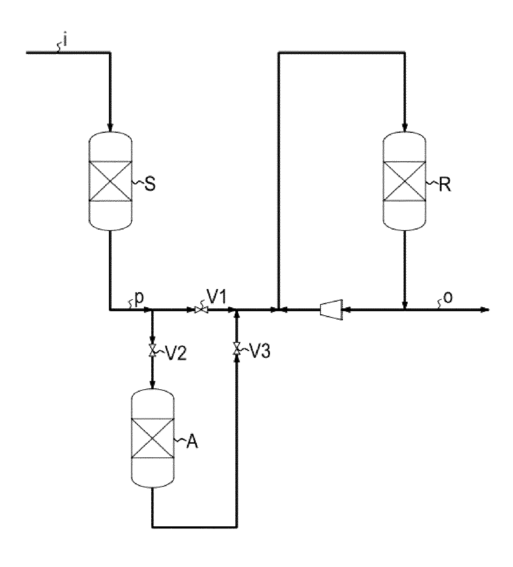

The reactor system according to the invention has a layout as shown on the appended figure. The inlet gas stream (i) is passed through a sulfur guard S and proceeds either directly to a reactor R, preferably a methanation reactor, or to an adsorption vessel A containing an adsorbent selective for adsorbing a reactor purge gas. The valve V1 is normally open, while the valves V2 and V3 are closed. This way the process gas stream (p) will proceed directly from the sulfur guard outlet to the reactor R. If the valve V1 is closed, while the valves V2 and V3 are opened, the process gas stream will pass through the adsorption vessel A, which can be a PSA unit or any other device containing an adsorbent as used in connection with PSA units. As mentioned above, the adsorption vessel A may advantageously be placed upstream instead of downstream the sulfur guard S. This way a temperature "runaway" or temperature rise in the sulfur guard can be avoided because of a formation of by-products in the stagnant low-flow gas.

The adsorption vessel may be preceded by a cooler (not shown) in order to increase the capacity of the vessel.

In the reactor system according to the invention, said process gas preferably is a synthesis gas comprising carbon oxides and hydrogen.

Preferably the adsorption vessel in the reactor system of the invention contains a carbon monoxide and methane selective adsorbent, such as Sylobead®. A "heat-sink" can be placed upstream the adsorption vessel to lower the temperature of the gas, whereby the capacity of the adsorbent may increase. The heat-sink may consist of an inert material, or it may be the adsorbent itself, most preferably with high heat capacity. During normal operation, when the adsorbent system is not in use, the heat sink and the adsorbent are cooled by the surroundings or alternatively cooled by an inert gas, such as nitrogen. When the plant is tripped or shut down, gas at an elevated temperature is led through the heat sink and adsorbent system, where the heat sink adsorbs the heat before cooled gas enters the adsorbent.

Sylobead® is a brand of molecular sieve and silica gel products originally developed to remove acid gas components such as CO₂, H₂S and other sulfur compounds from raw natural gas and recover by-products, such as condensates and natural gas liquids, from the gas. It is very well suited for use as a CO and CH₄ selective adsorbent in the adsorption vessel of the reactor system of the invention.

The adsorption vessel can also be used to simplify the reduction procedure, if any catalyst should need that. The reduction can be carried out by using syngas (i.e. CO + H₂). However, that procedure is simpler if the carbon monoxide can be removed. The reason why it is simpler is that CO₂ build-up can be avoided because CO₂ is formed when CO is used as a reducing agent.

Steam may be added to the hydrogen-rich gas downstream the adsorption vessel A to increase the cooling potential of the gas (mass/heat capacity).

In the reactor system according to the invention, the reactor R comprises a catalytically active material which is sensitive to the gas composition contacting the catalytically active material. The catalytically active material is preferably a material, which is active in the methanation process.

The process according to the invention for trips or shut-down of a reactor system comprises the following steps:
a) directing a process gas through an adsorption vessel arranged in parallel to the normal piping to separate methane and carbon monoxide from the gas, thereby decreasing the risk of carbon formation in a downstream reactor, and
b) adjusting the process conditions to provide a shut-down or trips state in the system.

The invention is illustrated further by the following examples:

### Example 1

This example shows the normal situation under operation of the system with a carbon limit of around 700°C. The individual streams in the system have the compositions as indicated in Table 1:

**Table 1**

| Stream | i | p | o |
|---|---|---|---|
| Composition | mole % | mole % | mole % |
| Ar | 0.1 | 0.2 | 0.2 |
| CH₄ | 15.0 | 45.8 | 45.8 |
| CO | 20.0 | 3.0 | 3.0 |
| CO₂ | 1.0 | 4.2 | 4.2 |
| H₂ | 63.9 | 25.8 | 25.8 |
| N₂ | 0.1 | 0.2 | 0.2 |
| H₂O | | 21.0 | 21.0 |

where "o" is the outlet gas stream after the reactor R.

### Example 2

This example shows the shut-down situation under operation of the system without PSA and with a carbon limit of around 535°C. The individual streams in the system have the compositions as indicated in Table 2:

**Table 2**

| Stream | i | p | o |
|---|---|---|---|
| Composition | mole % | mole % | mole % |
| Ar | 0.1 | 0.1 | 0.1 |
| CH₄ | 15.0 | 29.5 | 29.5 |
| CO | 20.0 | 11.0 | 11.0 |
| CO₂ | 1.0 | 3.5 | 3.5 |
| H₂ | 63.9 | 46.9 | 46.9 |
| N₂ | 0.1 | 0.1 | 0.1 |
| H₂O | | 8.9 | 8.9 |

### Example 3

This example shows the shut-down situation under operation of the system with PSA and with a carbon limit of around 885°C. The individual streams in the system have the compositions as indicated in Table 3:

**Table 3**

| Stream | i | V2 | V3 | p | o |
|---|---|---|---|---|---|
| Composition | mole % | mole % | mole % | mole % | mole % |
| Ar | 0.1 | 0.1 | 0.0 | 0.1 | 0.1 |
| CH₄ | 15.0 | 15.0 | 6.0 | 15.2 | 15.2 |
| CO | 20.0 | 20.0 | 8.0 | 1.3 | 1.3 |
| CO₂ | 1.0 | 1.0 | 0.4 | 0.3 | 0.3 |
| H₂ | 63.9 | 63.9 | 85.5 | 74.7 | 74.7 |
| N₂ | 0.1 | 0.1 | 0.0 | 0.0 | 0.0 |
| H₂O | | | | 8.4 | 8.4 |

## Claims

1. A reactor system comprising
- an inlet line (i) for a process gas,
- a sulfur guard (S),
- an adsorption vessel (A) containing an adsorbent selective for adsorbing a reactor purge gas, and
- an outlet line (o) for the product gas stream from a reactor (R),
wherein the adsorption vessel is installed either upstream or downstream the sulfur guard, in parallel to the normal piping, to be used in case of trips or shut-down.

2. Reactor system according to claim 1, wherein the adsorption vessel is based on the use of pressure swing adsorption (PSA).

3. Reactor system according to claim 1 or 2, wherein the adsorption vessel, arranged in parallel to the normal piping, is activated by closing the valve V1 on the normal piping and opening the two valves V2 and V3 on the parallel piping.

4. Reactor system according to any of the claims 1-3, wherein the reactor is a methanation reactor.

5. Reactor system according to any of the claims 1-4, wherein two adsorption vessels arranged in parallel are used, such that one vessel is regenerated, while the other is in use.

6. Reactor system according to any of the claims 1-5, wherein the adsorption vessel is preceded by a cooler in order to increase the capacity of the vessel.

7. Reactor system according to any of the claims 1-6, wherein a heat-sink is placed upstream the adsorption vessel to lower the gas temperature, thereby increasing the capacity of the adsorbent.

8. Reactor system according to claim 7, wherein the heat-sink consists of an inert material or is the adsorbent itself.

9. Reactor system according to claim 1, wherein the adsorption vessel is installed upstream the sulfur guard to avoid a temperature "runaway" or temperature rise by formation of by-products in the stagnant low-flow gas.

10. Reactor system according to any of the claims 1-9, wherein steam is added to the hydrogen-rich gas downstream the adsorption vessel A to increase the cooling potential of the gas (mass/heat capacity).

11. Reactor system according to any of the claims 1-10, wherein the adsorption vessel is regenerated by relieving the pressure to flare.

12. A process for trips or shut-down of a reactor system according to any of the claims 1-11, said process comprising the following steps:
a) directing a process gas through an adsorption vessel arranged in parallel to the normal piping to separate methane and carbon monoxide from the gas, thereby decreasing the risk of carbon formation in a downstream reactor, and
b) adjusting the process conditions to provide a shut-down or trips state in the system.
